# EUROPÄISCHE PATENTANMELDUNG

(11) **EP 2 151 208 A2**
(43) Veröffentlichungstag der Anmeldung: **10.02.2010**
(21) Anmeldenummer: 09010169.2
(22) Anmeldetag: 06.08.2009
(51) Int. Cl.: A61B 17/72

(54) **Distraktionsvorrichtung**

(30) Priorität: 06.08.2008 DE 102008036689
(71) Anmelder: Feld, Christoph M., 57299 Burbach (DE)
(72) Erfinder: Feld, Christoph M., 57299 Burbach (DE)
(74) Vertreter: Thul, Stephan

(57) **Zusammenfassung**

Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel, welcher zwei axial auseinanderfahrbare, jeweils an einem der beiden Knochenteile befestigbare Teile aufweist, mit einer eine Welle antreibenden Antriebseinheit und mit einer Vorrichtung zur Umsetzung der Rotationsbewegung der Antriebswelle in eine relative Axialbewegung der beiden Teile des Marknagels zueinander, wobei zur Verbesserung der Funktionssicherheit und der Verringerung der Baugröße bei gleichzeitig hohem Wirkungsgrad zur Umsetzung der Rotationsbewegung in eine Axialbewegung ein Kugel- oder Rollengewindetrieb mit Kugel- bzw. Rollenrückführung vorgesehen ist.

## Beschreibung

Die vorliegende Erfindung betrifft eine Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel, welcher zwei axial auseinanderfahrbare, jeweils an einem der beiden Knochenteile befestigbare Teile aufweist, mit einer eine Welle antreibenden Antriebseinheit und mit einer Vorrichtung zur Umsetzung der Rotationsbewegung der Antriebswelle in eine relative Axialbewegung der beiden Teile des Marknagels zueinander.

Die Verwendung einer Distraktionsvorrichtung mit Marknagel ist aus der DE 39 21 972 C2 bekannt. Diese Vorrichtung dient insbesondere zur Verlängerung von Röhrenknochen oder Defektüberbrückung nach Trümmerbrüchen, einer Knochenentzündung oder nach Entfernen von Tumoren im Bereich langer Röhrenknochen.

Die Funktionsweise des bekannten Marknagels ist derart, dass durch Auseinanderbewegen der beiden Teile des Marknagels die beiden Teile des Knochens langsam auseinander bewegt werden, wobei die sich dadurch bildende Lücke zwischen den beiden Knochenenden aufgrund des langsamen Vorschubes fortlaufend durch sich neu bildende Knochensubstanzen überbrückt wird. Auf diese Weise können Knochen nicht nur verlängert werden, indem Knochen nach Aufbohren und Einführen des Marknagels in den Markraum durchtrennt und anschließend auseinander gezogen werden, sondern es können auch Knochenlücken überbrückt werden, indem ein von einem der Lücke benachbarten Knochenende abgetrenntes Knochenstück zum anderen Ende der Lücke bewegt wird. Entsprechend kann auch das Ende eines Knochenstumpfes, also ein Knochen mit fehlendem Knochenende, verlängert werden.

Der Erfindung liegt die Aufgabe zugrunde, eine Distraktionsvorrichtung der eingangs genannten Art anzugeben, die sich durch eine hohe Funktionssicherheit auszeichnet und eine möglichst geringe Baugröße mit zugleich hohem Wirkungsgrad der Antriebsvorrichtung verbindet.

Diese Aufgabe wird dadurch gelöst, dass zur Umsetzung der Rotationsbewegung in eine Axialbewegung ein Kugel- oder Rollengewindetrieb mit Kugel- bzw. Rollenrückführung vorgesehen ist.

Die Verwendung eines Kugel- oder Rollengewindetriebes zur Umsetzung der Rotationsbewegung in eine Axialbewegung hat sich als besonders vorteilhaft herausgestellt. Nicht nur kann dadurch eine sehr hohe Genauigkeit gewährleistet werden, sondern auch eine hohe Funktionssicherheit, da durch diesen Trieb eine nahezu reibungslose Bewegung ermöglicht ist.

Besonders bevorzugt ist der Marknagel teleskopartig ausgebildet und weist einen Aufnahmeteil sowie mindestens einen ausfahrbaren Teil auf. Die Relativbewegung der beiden Knochenteile kann dadurch in besonders günstiger Weise und auch sehr positionsgenau gewährleistet werden.

Bevorzugt ist die Spindel des Gewindetriebs im Aufnahmeteil gelagert und die Mutter des Gewindetriebes mit dem ausfahrbaren Teil verbunden. Dies gewährleistet eine besonders hohe Funktionssicherheit.

Nach einer weiteren Ausgestaltung der Erfindung nimmt der Aufnahmeteil auch den Motor auf. Dadurch ergibt sich eine kompakte und leicht handhabbare Ausgestaltung.

Nach noch einer Ausgestaltung der Erfindung besteht die Spindel aus Metall und die Mutter aus Keramik. Diese Hybridausgestaltung hat den Vorteil, dass nicht Metall gegen Metall läuft. Die Lagerung kann dadurch schmierungsfrei ausgeführt werden.

Der ausfahrbare Teil des Teleskopmarknagels ist bevorzugt über eine Gleithülse, insbesondere aus medizinischem Kunststoff, in der Aufnahme gelagert. Damit kann eine exakte Führung des ausfahrbaren Teils zusätzlich zur Mutter gewährleistet werden.

Durch eine Abdichtung des ausfahrbaren Teils gegenüber der Aufnahme kann die Bioverträglichkeit und Funktionssicherheit der Vorrichtung verbessert werden. Bevorzugt ist die Dichtung dabei mit einem Sonderprofil ausgebildet, wodurch sich die Abdichtung weiter verbessern lässt.

Zwischen Aufnahmeteil und ausfahrbarem Teil des Teleskopmarknagels ist nach einer weiteren Ausgestaltung der Erfindung ein verdrehsicheres Führungssystem vorhanden, wobei das Führungssystem bevorzugt durch eine relativ zur Spindelachse positionierbare Hülse gebildet ist. Durch Verdrehen der Hülse kann ein Klemmen der Führung verhindert werden.

Die Führung kann des Weiteren durch zwei einander gegenüberliegende positive Führungen, so genannte Felder, und zwei entsprechend angeordnete negative Führungen, so genannte Züge, gebildet sein. Nach einer anderen Ausgestaltung der Erfindung kann die Führung durch drei Felder und drei entsprechende Züge gebildet sein, die insbesondere um ca. 100 bis 120° in Umfangsrichtung zueinander versetzt angeordnet sind. Derartige Führungen haben sich als besonders sicher erwiesen.

Nach einer weiteren Ausgestaltung der Erfindung, die auch für sich beansprucht wird, ist die Spindel flexibel ausgebildet. Dadurch ist es möglich, die Spindel mit einer Krümmung auszubilden, wodurch ein Einsatz der Distraktionsvorrichtung auch bei gekrümmten Knochen möglich oder verbessert ist. Die Krümmung kann dabei bevorzugt patientenindividuell eingestellt sein.

Zur Bewirkung der Krümmung der Spindel ist nach einer Ausgestaltung der Erfindung der Aufnahmeteil entsprechend gebogen ausgebildet. Die Krümmung ergibt sich dann durch die Führung des ausfahrbaren Teils im Aufnahmeteil.

Als Antrieb der Distraktionsvorrichtung dient bevorzugt ein Elektromotor. Der Motor kann dabei bevorzugt induktiv, insbesondere durch Hochfrequenzeinkoppeln, mit Energie versorgbar sein. Alternativ oder zusätzlich kann der Motor über einen implantierbaren Speicher mit Energie versorgbar sein. Der implantierbare Speicher ist dabei bevorzugt von außen aufladbar, insbesondere durch induktive Einkopplung. Damit kann jeweils gewährleistet werden, dass die Distraktionsvorrichtung über einen längeren Zeitraum eingesetzt werden kann, ohne dass eine erneute Operation des Patienten erforderlich ist, um einen Energiespeicher auszutauschen.

Nach einer weiteren Ausgestaltung der Erfindung ist eine implantierbare Kraft- und/oder Wegmesseinrichtung für die Bewegung der Distraktionsvorrichtung vorgesehen. Damit kann die Distraktion überwacht und gesteuert werden.

Nach einer weiteren Ausgestaltung der Erfindung, die ebenfalls für sich beansprucht wird, ist mindestens ein Teil der Vorrichtung mit einem körperverträglichen, porenlos dichten Material, insbesondere Titan, überzogen. Durch diesen dichten Überzug kann auch körperunverträgliches Material für bestimmte Teile oder die gesamte Vorrichtung verwendet werden. Dies hat den Vorteil, dass die Materialauswahl für die betroffenen Teile unabhängig von der Körperverträglichkeit vorgenommen werden kann, da diese durch den Überzug gewährleistet wird.

Nach einer weiteren Ausgestaltung der Erfindung umfasst die Steuerung zwei verschiedene Modi, nämlich einen Patientenmodus zum Auslösen eines vom Arzt eingestellten Vorschubs und einen insbesondere passwortgeschützten Arztmodus zum Einstellen eines Vorschubs. Die Handhabung der Vorrichtung ist dadurch einfach und sicher.

Zur Überwachung eines ordnungsgemäß stattgefundenen Vorschubs kann außerdem nach einer weiteren Ausgestaltung der Erfindung eine Anzeige, insbesondere optische Anzeige, beispielsweise durch LED, vorgesehen sein. Der Patient kann dadurch überprüfen, ob die Vorrichtung einwandfrei funktioniert hat.

Nach einer weiteren Ausgestaltung der Erfindung kann die Steuerung und/oder der Energiespeicher als separat implantierbare Einheit ausgebildet sein. Die Distraktionsvorrichtung selbst kann dadurch noch kleiner ausgebildet sein, so dass ein Einsatz auch bei kleinen Knochen möglich wird.

Nach noch einer Ausgestaltung der Erfindung, die ebenfalls für sich beansprucht wird, sind Mittel vorgesehen, durch welche der Vorschub automatisch auslösbar ist, insbesondere abhängig vom Schlaf-Wach-Rhythmus des Patienten. Damit kann ein Vorschub beispielsweise in Schlafphasen des Patienten ausgelöst werden, so dass der Patient noch weniger belastet wird.

Bevorzugt ist es des Weiteren, wenn Mittel vorgesehen sind, durch welche alle im System auftretenden Kräfte wie Axial-, Radial-, Scher- und Torsionskräfte erfassbar und nach außen übertragbar sind. Die Funktion des Systems kann dadurch überwacht und sichergestellt werden.

Nach noch einer auch für sich beanspruchten Ausgestaltung der Erfindung ist die Außenhülle der Distraktionsvorrichtung als Antenne für die Datenübertragung und/oder Energieeinspeisung ausgebildet. Eine separate Antenne kann dadurch vermieden werden, insbesondere eine außen angeordnete Antenne, die über ihre Verbindung mit dem Marknagel einen Infektionsherd bildet.

Ausführungsbeispiele der Erfindung sind in den Zeichnungen dargestellt und werden nachfolgend beschrieben. Es zeigen, jeweils in schematischer Darstellung,
- Fig. 1: einen Schnitt durch einen erfindungsgemäßen Marknagel,
- Fig. 2: einen Querschnitt durch eine erste Ausführungsform einer Gleithülse und
- Fig. 3: einen Querschnitt durch eine zweite Ausführungsform einer Gleithülse.

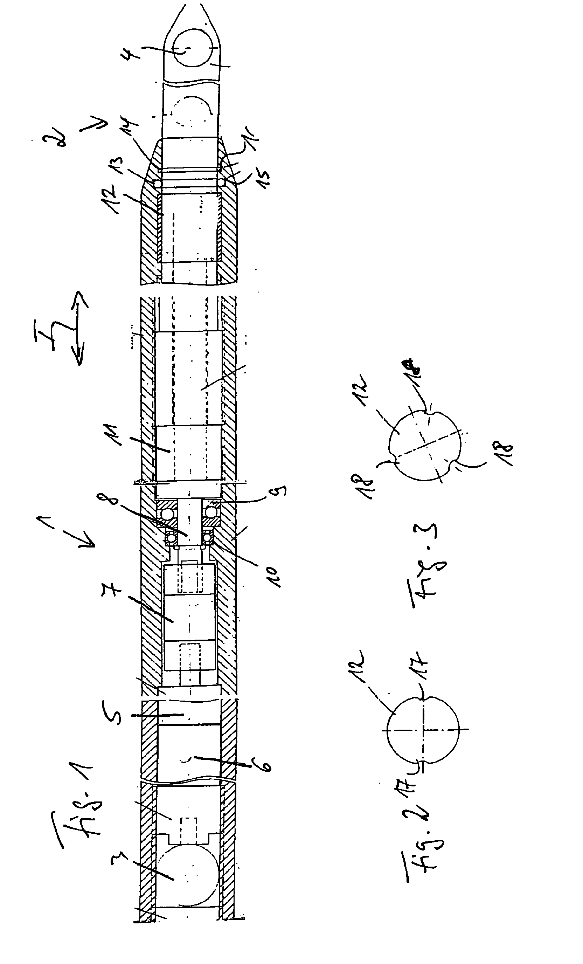

Die in Fig. 1 dargestellte Distraktionsvorrichtung ist als Teleskopmarknagel ausgebildet und weist einen Aufnahmeteil 1 und einen ausfahrbaren Teil 2 auf, die jeweils mit einer Nagelbohrung 3, 4 zur Befestigung an einem Knochen oder Knochenteil versehen sind. Im Aufnahmeteil 1 ist ein Elektromotor 5 mit Steuerung 6 und Kupplung 7 angeordnet. Über die Kupplung 7 ist der Motor 5 mit einer Spindel 8 verbunden, die über eine Axiallagerung 9 und eine Radiallagerung 10 im Aufnahmeteil 1 gelagert ist.

Die Spindel 8 ist Teil eines Kugelgewindetriebes, dessen anderer Teil durch eine Mutter 11 mit hier nicht erkennbaren Kugeln gebildet ist. Der Kugelgewindetrieb ist im Prinzip in bekannter Weise ausgebildet. Mit der Mutter 11 des Kugelgewindetriebes verbunden ist der ausfahrbare Teil 2 des Marknagels. Durch Verdrehen der Spindel 8 kann so über die Mutter 11 der ausfahrbare Teil 2 gegenüber dem Aufnahmeteil 1 ein- und ausgefahren werden.

In seinem einen Endbereich ist im Aufnahmeteil 1 zudem eine Gleithülse 12 vorgesehen, in welcher der ausfahrbare Teil 2 gleitend geführt ist. Die Gleithülse besteht bevorzugt aus medizinischem Kunststoff. Schließlich sind am Ende des Aufnahmeteils 1 zwei Dichtungen 13 und 14 vorgesehen, die in Nuten 15 und 16 des Aufnahmeteils 1 eingelegt sind.

Wie in den Fig. 2 und 3 dargestellt, ist die Gleithülse 12 zusätzlich als Verdrehsicherung ausgebildet. Hierfür ist der ausfahrbare Teil 2 an seinem Außenumfang mit zwei Zügen 17 (Fig. 2) oder drei Zügen 18 (Fig. 3) versehen. Die Gleithülse 12 weist dementsprechend auf ihrem Innenumfang zwei oder drei Felder auf. Zudem ist die Gleithülse 12 bevorzugt innerhalb des Aufnahmeteils 1 in Umfangsrichtung in unterschiedlichen Positionen festlegbar. Dadurch kann die Verdrehsicherung über die Züge 17, 18 und die Felder verändert werden, um ein Verklemmen zu vermeiden.

Die Spindel 8 besteht bevorzugt aus Metall, während die Mutter 11 bevorzugt aus Keramik besteht. Dadurch kann auf eine Schmierung verzichtet werden. Die Dichtungen 13, 14 weisen ein Sonderprofil auf. Die Spindel 8 kann auch flexibel ausgebildet sein, um eine Krümmung zu ermöglichen. Um eine solche Krümmung zu realisieren, kann der Aufnahmeteil 1 entsprechend gekrümmt ausgebildet sein. Über die Führung durch die Mutter 11 und die Gleithülse 12 ergibt sich die gewünschte Krümmung, um in gekrümmten Knochen besser arbeiten zu können.

Zur Energieversorgung des Motors 5 kann ein hier nicht dargestellter Energiespeicher vorgesehen sein, der entweder ebenfalls innerhalb des Aufnahmeteils 1 angeordnet werden oder separat implantierbar sein kann. Der Energiespeicher kann von außen induktiv aufladbar sein. Alternativ oder zusätzlich kann auch der Motor 5 von außen unmittelbar mit Energie versorgbar sein. In beiden Fällen erfolgt die Energieeinkopplung bevorzugt über Hochfrequenz. Des Weiteren kann der Marknagel eine ebenfalls nicht dargestellte Kraft- und/oder Wegmesseinrichtung aufweisen sowie Mittel zur Übertragung der festgestellten Kräfte und Wege nach außen. Über eine ebenfalls nicht dargestellte Anzeigevorrichtung, beispielsweise LED, kann eine ordnungsgemäße Funktion des Marknagels nach außen angezeigt werden. Die Steuerung 6 kann zwei Modi, nämlich einen Patientenmodus und einen Arztmodus umfassen. Über den Patientenmodus kann der Patient einen Vorschub auslösen. Es kann aber auch eine automatische Auslösung vorgesehen sein, insbesondere abhängig vom Schlaf/Wach-Rhythmus des Patienten. Zur Energie- und Datenübertragung kann die Außenhülle des Marknagels, insbesondere des Aufnahmeteils 1 als Antenne ausgebildet sein. Teile des Marknagels können außerdem mit einer porenlos dichten Titanbeschichtung versehen sein, um eine unabhängige Materialwahl für diese Teile zu ermöglichen.

### Bezugszeichenliste

- 1: Aufnahmeteil
- 2: ausfahrbarer Teil
- 3: Nagelbohrung
- 4: Nagelbohrung
- 5: Motor
- 6: Steuerung
- 7: Kupplung
- 8: Spindel
- 9: Axiallager
- 10: Radiallager
- 11: Mutter
- 12: Gleithülse
- 13: Dichtung
- 14: Dichtung
- 15: Nut
- 16: Nut
- 17: Zug
- 18: Zug
- I: Ausfahrrichtung

## Patentansprüche

1. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung der beiden Teile (1, 2) des Marknagels zueinander,
**dadurch gekennzeichnet, dass**
zur Umsetzung der Rotationsbewegung in eine Axialbewegung ein Kugel- oder Rollengewindetrieb (8, 11) mit Kugel- bzw. Rollenrückführung vorgesehen ist.

2. Vorrichtung nach Anspruch 1,
**dadurch gekennzeichnet, dass**
der Marknagel teleskopartig ausgebildet ist mit einem Aufnahmeteil (1) und mindestens einem ausfahrbaren Teil (2).

3. Vorrichtung nach Anspruch 2,
**dadurch gekennzeichnet, dass**
die Spindel (8) des Gewindetriebs im Aufnahmeteil (1) gelagert ist und dass die Mutter (11) des Gewindetriebs mit dem ausfahrbaren Teil (2) verbunden ist,
und/oder dass
der Aufnahmeteil (1) auch den Motor (5) aufnimmt,
und/oder dass
der ausfahrbare Teil (2) in der Aufnahme (1) über eine Gleithülse (12), insbesondere aus medizinischem Kunststoff, gelagert ist, und/oder dass
der ausfahrbare Teil (2) gegenüber der Aufnahme (1) abgedichtet ist, wobei insbesondere die Dichtungen (13, 14) mit einem Sonderprofil ausgestattet sind.

4. Vorrichtung nach einem der vorhergehenden Ansprüche,
**dadurch gekennzeichnet, dass**
die Spindel (8) aus Metall und die Mutter (11) aus Keramik besteht.

5. Vorrichtung nach einem der Ansprüche 2 bis 4,
**dadurch gekennzeichnet, dass**
zwischen Aufnahmeteil (1) und ausfahrbarem Teil (2) ein verdrehsicheres Führungssystem (17, 18) vorhanden ist, wobei das Führungssystem bevorzugt durch eine relativ zur Spindelachse positionierbare Hülse (12) gebildet ist.

6. Vorrichtung nach Anspruch 5,
**dadurch gekennzeichnet, dass**
das Führungssystem zwei einander gegenüberliegende positive Führungen, so genannte Felder, und zwei entsprechend angeordnete negative Führungen (17), so genannte Züge, aufweist,
oder
drei Felder und drei Züge (18) vorgesehen sind, die insbesondere um ca. 100 bis 120° in Umfangsrichtung zueinander versetzt angeordnet sind.

7. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung
der beiden Teile (1, 2) des Marknagels zueinander, insbesondere nach einem der vorhergehenden Ansprüche,
**dadurch gekennzeichnet, dass**
die Spindel (8) flexibel ist.

8. Vorrichtung nach Anspruch 7,
**dadurch gekennzeichnet, dass**
die Spindel (8) eine Krümmung aufweist, insbesondere patientenindividuell eingestellt, wobei der Aufnahmeteil (1) bevorzugt entsprechend der gewünschten Spindelkrümmung gebogen ist.

9. Vorrichtung nach einem der vorhergehenden Ansprüche,
**dadurch gekennzeichnet, dass**
ein Elektromotor (5) als Antrieb dient, wobei
der Motor (5) bevorzugt induktiv, insbesondere durch Hochfrequenzeinkopplung, mit Energie versorgbar ist
und/oder
der Motor (5) über einen implantierbaren Speicher mit Energie versorgbar ist, wobei der implantierbare Speicher bevorzugt von außen aufladbar ist, insbesondere durch induktive Ankopplung.

10. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung der beiden Teile (1, 2) des Marknagels zueinander, insbesondere nach einem der vorhergehenden Ansprüche,
**dadurch gekennzeichnet, dass**
eine implantierbare Kraft- und/oder Wegmesseinrichtung für die Bewegung der Distraktionsvorrichtung vorgesehen ist.

11. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung der beiden Teile (1, 2) des Marknagels zueinander, insbesondere nach einem der vorhergehenden Ansprüche,
**dadurch gekennzeichnet, dass**
mindestens ein Teil der Vorrichtung mit einem körperverträglichen, porenlos dichten Material, insbesondere Titan, überzogen ist.

12. Vorrichtung nach einem der vorhergehenden Ansprüche,
**dadurch gekennzeichnet, dass**
die Steuerung zwei verschiedene Modi umfasst, nämlich einen Patientenmodus zum Auslösen eines vom Arzt eingestellten Vorschubs und einen insbesondere passwortgeschützten Arztmodus zum Einstellen eines Vorschubs,
und/oder dass
eine insbesondere optische Anzeige vorgesehen ist zum Anzeigen eines ordnungsgemäß stattgefundenen Vorschubs,
und/oder dass
die Steuerung und/oder der Energiespeicher als separat implantierbare Einheit ausgebildet sind.

13. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung der beiden Teile (1, 2) des Marknagels zueinander, insbesondere nach einem der vorhergehenden Ansprüche,
**dadurch gekennzeichnet, dass**
Mittel vorgesehen sind, durch welche der Vorschub automatisch auslösbar ist, insbesondere abhängig vom Schlaf-/Wach-Rhythmus des Patienten.

14. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung der beiden Teile (1, 2) des Marknagels zueinander, insbesondere nach einem der vorhergehenden Ansprüche,
**dadurch gekennzeichnet, dass**
Mittel vorgesehen sind, durch welche alle im System auftretenden Kräfte erfassbar und nach außen übertragbar sind.

15. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1, 2), welcher zwei axial auseinanderfahrbare, jeweils an einem der beiden Knochenteile befestigbare Teile (1, 2) aufweist, mit einer eine Welle (8) antreibenden Antriebseinheit (5) und mit einer Vorrichtung zur Umsetzung der Rotationsbewegung der Antriebswelle (8) in eine relative Axialbewegung der beiden Teile (1, 2) des Marknagels zueinander, insbesondere nach einem der vorhergehenden Ansprüche,
**dadurch gekennzeichnet, dass**
die Außenhülle der Distraktionsvorrichtung als Antenne für die Datenübertragung und/oder Energieeinspeisung ausgebildet ist.
